# FASCICULE DE BREVET EUROPEEN

(11) **EP 2 547 291 B1**
(45) Date de publication et mention de la délivrance du brevet: **23.04.2014**
(21) Numéro de dépôt: 11716284.2
(22) Date de dépôt: 16.03.2011
(51) Int. Cl.: A61F 2/38

(54) **PROTHESE DU GENOU AVEC PLAQUE MENISCALE MIXTE**
KNIEPROTHESE MIT GEMISCHTER MENISKUSSCHEIBE
KNEE PROSTHESIS HAVING A MIXED MENISCAL PLATE

(30) Priorité: 16.03.2010 FR 1001056
(43) Date de publication de la demande: 23.01.2013
(73) Titulaire: Implanet, 33650 Martillac (FR)
(72) Inventeur: LE COUEDIC, Régis, F-33800 Bordeaux (FR); PASQUET, Denis, F-33360 Quinsac (FR)
(74) Mandataire: Benech, Frédéric
(86) Numéro de dépôt international: PCT/FR2011/000148
(87) Numéro de publication internationale: WO 2011/114024

(56) Documents cités:
- EP-A2- 0 732 091
- FR-A1- 2 837 093
- US-A1- 2006 161 259
- US-A1- 2008 114 462

## Description

La présente invention concerne une prothèse du genou comprenant une pièce fémorale, un plateau tibial et une plaque méniscale intermédiaire.

Elle trouve une application particulièrement importante, bien que non exclusive, dans le domaine des prothèses du genou dites totales.

On sait que les prothèses du genou sont en général de type tricompartimental.

En d'autres termes, ce sont des prothèses comprenant d'un côté deux éléments, permettant de remplacer les deux parties fémorale et tibiale de l'articulation entre le fémur et le tibia, et de l'autre côté un élément pour former la surface intermédiaire de frottement de la rotule.

La structure prothétique complète ou totale comporte donc une pièce ou plan fémoral, un plateau ou plan tibial et une plaque méniscale appelée souvent insert tibial. Elle est complétée par un implant rotulien, qui n'ayant pas de rôle dans le cadre de la présente invention ne sera plus mentionné par la suite.

De façon connue en elle-même, le plateau tibial coopère avec la pièce fémorale via la plaque méniscale, pour réaliser ainsi les différents mouvements de l'articulation du genou.

Dès maintenant il convient de remarquer que, de façon à pouvoir s'adapter aux différentes morphologies des patients, tous ces implants se doivent d'exister en plusieurs tailles et épaisseurs.

Concernant l'utilisation de ces prothèses, deux techniques président essentiellement à la reconstruction de l'articulation du genou.

La première technique consiste à utiliser une plaque méniscale fixe par rapport au plateau tibial.

La deuxième technique consiste à utiliser des plaques méniscales dites mobiles, par opposition aux plaques méniscales fixes.

Ces.plaques mobiles ont pour but d'aider à mieux reproduire la cinématique de l'articulation en réduisant les mouvements paradoxaux dus à la pièce fémorale.

Pour permettre la mise en oeuvre de ces deux techniques, le chirurgien doit donc disposer de plaques méniscales pour chaque taille et pour chaque technique possible et ce en plusieurs épaisseurs.

De plus et quelques soient les plaques méniscales utilisées, qu'elles soient fixes ou mobiles, une partie des chirurgiens considère qu'il est important de conserver le ligament croisé postérieur, lorsque cela est possible, pour limiter les déplacements antérieurs du fémur par rapport au tibia.

L'autre partie des chirurgiens est par contre partisane d'une ablation de ce ligament, préférant confier la limitation du déplacement antérieur fémoral au dessin particulier de la plaque méniscale.

Il faut ainsi des plaques fixes avec croisé, des plaques fixes sans croisé, des plaques mobiles avec croisé et des plaques mobiles sans croisé ce qui multiplie encore le nombre de plaques. A noter que « sans croisé » est équivalent à « postéro-stabilisé ».

Le chirurgien doit alors choisir la plaque méniscale qui assurera une reconstruction optimale de l'articulation du patient en fonction de la stabilité et de l'amplitude des mouvements recherchés pour le patient concerné.

Les prothèses de l'art antérieur présentent dès lors notamment un inconvénient important. Elles nécessitent de prévoir un très grand nombre de pièces fémorales, de plateaux tibiaux et de plaques méniscales, pour parer à toutes les éventualités.

En d'autres termes, une gamme d'implants de genou qui intègre les deux types de plaques méniscales, à savoir les plaques mobiles et les plaques fixes, et ce tout en permettant de choisir entre la conservation du ligament postérieur ou son sacrifice, est nécessairement composée d'un nombre élevé de pièces.

Par exemple une gamme de prothèses de l'art antérieur constituée de huit tailles de pièces fémorales sur chaque côté, pour chaque type, soit trente deux pièces fémorales au total, trois épaisseurs de plaques méniscales pour chaque taille et chaque type et enfin seize tailles de plaques tibiales, nécessite de prévoir cent quarante quatre éléments différents.

On connaît (US 2006/0161259) des prothèses comprenant une pièce de fixation centrale prévues pour fonctionner après une ablation du ligament.

On connaît également (EP 0 732 091) des prothèses agencées pour fonctionner sans ablation avec jeu antéro-postérieur et possibilité de rotation.

De telles prothèses donnent lieu à des mouvements paradoxaux conduisant à des luxations de l'articulation et à des usures anormales de la pièce méniscale.

De plus aucune de ces prothèses ne permet de régler simultanément les deux cas cliniques.

Un des objets de la présente invention est de proposer une prothèse du genou répondant mieux que celles antérieurement connues aux exigences de la pratique, notamment en ce qu'elle autorise les mêmes possibilités physiques que les prothèses de l'art antérieur, mais nécessitent un nombre nettement plus faible d'éléments.

Ainsi pratiquement toutes les pièces constitutives d'une prothèse selon l'invention vont permettre de fonctionner dans un cas avec ablation du ligament et dans l'autre cas sans ablation du ligament.

Grâce à un des aspects de l'invention il va ainsi être possible de réduire les stocks et donc les investissements du fabricant d'implants, tout en augmentant la sécurité du patient par l'élimination de sources d'erreurs potentielles.

De plus, l'invention autorise un gain de temps opératoire et un apprentissage rapide de la gamme d'implants par les chirurgiens et les personnels de blocs opératoires.

Dans ce but, l'invention propose essentiellement une prothèse du genou comprenant une pièce fémorale reliée à un plateau tibial par une plaque méniscale intermédiaire munie d'une face supérieure, ladite pièce fémorale comportant un bouclier trochléen muni d'une face externe coopérant à frottement doux avec au moins une portée de guidage de forme complémentaire ménagée dans ladite face supérieure,
caractérisée en ce que la plaque méniscale intermédiaire et le plateau tibial sont reliés l'un à l'autre par l'intermédiaire de deux faces planes de jonction, à savoir une face de jonction tibiale et une face de jonction méniscale, la face de jonction tibiale étant plus grande que la face de jonction méniscale dans ses dimensions antéro-postérieures et médio latérale,
en ce que le plateau tibial comprend un pion central de pivotement ou au moins un doigt de clippage/indexage, ledit pion ou ledit doigt étant en saillie par rapport à la face de jonction tibiale,
et en ce que la face de jonction méniscale de la plaque méniscale comprend en combinaison un orifice borgne central propre à coopérer à frottement doux sur toute sa périphérie avec le pion de pivotement du plateau tibial dans un cas et un évidemment de blocage propre à s'encastrer avec le doigt d'indexage du plateau tibial dans l'autre, selon que le plateau tibial comporte un pion de pivotement ou un doigt d'indexage.

Ainsi, grâce à ce dessin particulier de la plaque méniscale, la même plaque méniscale que l'on peut également qualifier de plaque dite mixte, peut être indifféremment utilisée soit comme plaque fixe, soit comme plaque mobile, en fonction du plateau tibial choisi par le chirurgien, ce qui va réduire le stock des plaques méniscales par deux.

Dans le mode de réalisation plus particulièrement décrit ici, la face de jonction tibiale est plus grande que la face de jonction méniscale sur la totalité de ses dimensions parallèles à son axe de symétrie antéro-postérieur, ainsi que perpendiculairement à cet axe (dimensions médio-latérales).

En d'autres termes, lorsque la plaque méniscale est placée centrée, et dans sa position médiane sur le plateau tibial, la totalité de la surface de jonction méniscale s'inscrit entièrement à l'intérieur de la surface de jonction tibiale, qui présente un bord qui dépasse toujours, par exemple d'1 mm, de la périphérie de la surface de jonction méniscale, cette périphérie n'étant jamais en coïncidence à aucun endroit avec ledit bord dans cette position.

Cette disposition va d'une part autoriser de façon étonnante la standardisation des pièces aboutissant à l'invention, et d'autre part va permettre de protéger les ligaments et/ou parties molles appartenant et/ou adjacentes à l'articulation.

Avantageusement, les surfaces des faces planes de jonction sont en forme d'ellipse ou sensiblement en forme d'ellipse, tronquée sur un bord, par exemple sur 1/10^{ème} voir 1/20^{ème} de leur surface, parallèlement au grand axe de l'ellipse.

Les surfaces sont agencées pour permettre un pivotement en rotation de l'une par rapport à l'autre, de telle sorte que la périphérie externe courbe de la face de jonction méniscale en forme de portion d'ellipse, ou sensiblement en forme de portion d'éllipse, reste inscrite à l'intérieur de la face de jonction de la plaque tibiale, pour un angle de rotation entre les faces compris entre - 12° et + 12°, voir - 8° et + 8° et/ou - 4°et + 4°, par rapport à l'axe médio-latéral de la plaque méniscale, ou grand axe de l'ellipse.

Dans la pratique les mouvements du genou étant en effet physiologiquement limités en rotation de l'ordre de ± 4 à 5°, voir ± 7 à 8°, de telles dispositions permettent de préserver les partie molles sans être obligé de prévoir des butées spécifiques.

Il y a donc ainsi de façon inattendue protection naturelle des parties molles.

Par ailleurs, le fait que la périphérie externe (potentiellement agressive) de la plaque méniscale, ainsi toujours inscrite en fonctionnement à l'intérieur de la surface de la plaque tibiale, puisse pivoter en rotation autour d'un pion solidaire du plateau ou pièce tibiale, sans possibilité de glissement de la plaque méniscale par rapport au plateau tibial, ou au contraire être rigidement fixé à ladite pièce tibiale par des doigts, évite les portes à faux en position d'équilibre, créateurs de micro-contraintes entraînant une usure plus rapide et/ou des douleurs.

Dans des modes de réalisation particuliers, on a de plus recours à l'une et/ou à l'autre des dispositions suivantes :
- la face supérieure de la plaque méniscale est munie d'un pion et de deux portées de guidage ménagées dans la face supérieure de part et d'autre dudit pion, le bouclier trochléen de la face fémorale comportant une fente de guidage dudit pion ;
- le plateau tibial comprend au moins un doigt d'indexage, agencé pour s'encastrer dans l'évidemment de blocage de la plaque méniscale dans une position déterminée par rapport au plateau tibial ;
- le plateau tibial comprend deux doigts d'indexage postérieurs et un doigt d'indexage antérieur propre à coopérer avec deux évidements postérieurs et un évidemment antérieur en vis à vis de la plaque méniscale ;
- la face de jonction tibiale comporte un rebord périphérique formant doigt de clippage le long d'au moins une partie de la périphérie dudit plateau dans lequel s'encastre entièrement la face de jonction méniscale ;
- le plateau tibial comprend un pion central de pivotement, la plaque méniscale étant montée mobile en rotation par rapport au plateau tibial autour dudit pion ;
- le pion de la face supérieure de la plaque méniscale a une forme de bonnet phrygien ou de pouce ;
- le bonnet phrygien comporte une lèvre supérieure formant un léger ressaut agencé pour engendrer un mouvement de recul de la pièce fémorale en cas de décoaptation supérieure à 1 mm de celle-ci par rapport à la plaque méniscale.

Une telle disposition permet d'éviter une luxation antérieure de l'articulation prothétique ;
- la face de jonction méniscale comporte au moins un évidemment en épis par rapport à la périphérie de ladite face, agencé pour permettre la déconnexion de la plaque méniscale d'avec le plateau tibial. Cet évidement accessible latéralement par le chirurgien avec un instrument lui permet en effet de les déconnecter ou de les déclipser l'une de l'autre, en faisant bras de levier ;
- la zone postérieure au pion central de la plaque méniscale comporte une partie de renfort reliant les deux zones postéro-latérales de frottement articulaire ;
- la pièce fémorale et le plateau tibial sont en alliage métallique et la plaque méniscale est en matériau plastique polymère par exemple en polyéthylène.

L'invention propose également un kit de plusieurs prothèses telles que décrites ci-avant, caractérisé en ce qu'il comporte au moins un plateau tibial pour plaque méniscale fixe et au moins un plateau tibial pour plaque méniscale mobile, et un jeu de plaques méniscales correspondantes de plusieurs épaisseurs.

L'invention sera mieux comprise à la lecture de la description qui suit de modes de réalisation donnés à titre d'exemples non limitatifs.

La description se réfère aux figures qui l'accompagnent dans lesquelles :
Les figures 1A et 1B montrent en perspective, respectivement en vue de dessus et en vue de dessous les trois éléments, détachés les uns des autres, d'une prothèse selon un premier mode de réalisation de l'invention.
Les figures 2A et 2B montrent en perspective, respectivement en vue de dessus et en vue de dessous un deuxième mode de réalisation d'une prothèse selon l'invention, ici encore les éléments étant détachés les uns des autres.
Les figures 3A à 3D donnent respectivement en perspective axionométrique, en vue arrière, en coupe latérale et par-dessous, des vues de la plaque méniscale selon le mode de réalisation de l'invention plus particulièrement décrit ici.
Les figures 4A à 4C donnent respectivement en perspective axionométrique, en coupe latérale et de face, des vues du plateau tibial selon le mode de réalisation des figures 1.
Les figures 5A à 5C donnent respectivement en perspective axionométrique, en coupe latérale et de face, des vues du plateau tibial selon le mode de réalisation des figures 2.
Les figures 6A et 6B sont des vues, de dessous, en perspective et en plan, d'un mode de réalisation du plateau tibial selon l'invention.
Les figures 7A et 7B montrent schématiquement en vue de dessus les positions relatives de la face de jonction de la plaque méniscale, par rapport à la face de jonction du plateau tibial.

Dans la suite de la description on utilisera les mêmes numéros de référence pour désigner des éléments identiques.

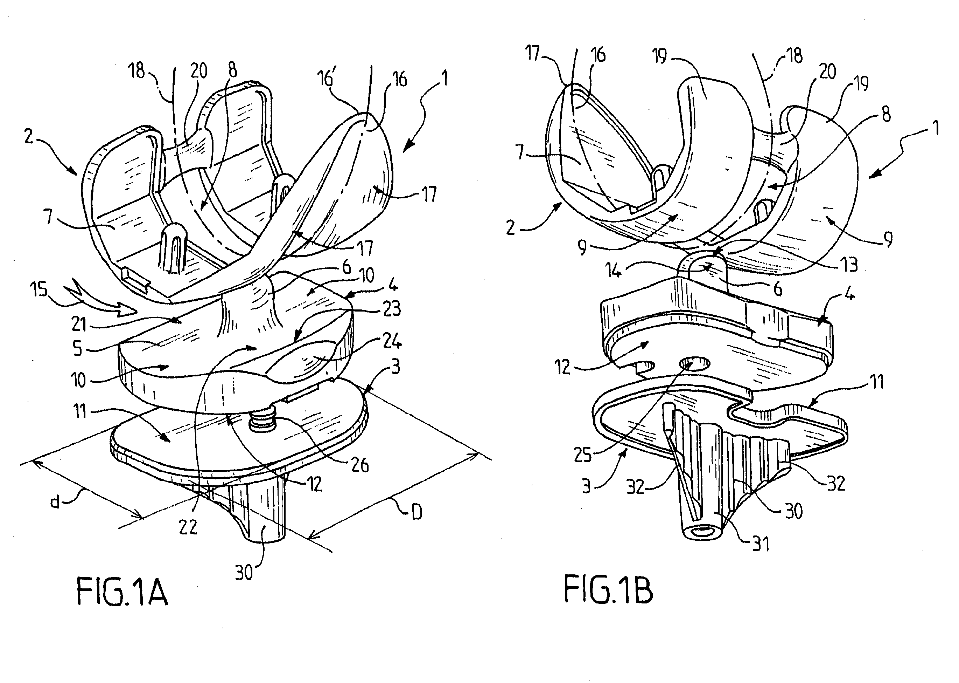

Les figures 1A et 1B montrent une prothèse 1 de genou comprenant une pièce fémorale 2 reliée à un plateau tibial 3 par une plaque méniscale 4 intermédiaire munie d'une face supérieure 5 comprenant un pion 6. La pièce fémorale 2 comporte un bouclier trochléen 7 muni d'une fente 8 de guidage du pion et une face externe 9 coopérant à frottement doux avec deux portées de guidage 10 de forme complémentaire, ménagées dans la face supérieure 5 de part et d'autre du pion.

La plaque méniscale intermédiaire 4 et le plateau tibial 3 sont reliés l'un à l'autre par l'intermédiaire de deux faces planes de jonction, à savoir une face 11 de jonction tibiale et une face 12 de jonction méniscale, la face 11 de jonction tibiale étant plus grande que la face 12 de jonction méniscale dans sa dimension antéro postérieure d et médio latérale D.

Le pion 6 présente une forme de bonnet phrygien ou de phalange supérieure de pouce, comportant une lèvre supérieure 13 formant un léger ressaut 14 agencé pour engendrer un mouvement de recul de la pièce fémorale en cas de décoaptation (flèche 15) supérieur à 1 mm de ladite pièce fémorale par rapport à la plaque méniscale.

Plus précisément, la pièce fémorale 2 comprend une première partie 16 en forme de langue se terminant légèrement en pointe 16', définissant la surface de révolution concave de la face externe 9 de glissement qui présente un rayon de courbure variable entre ladite première partie en forme de langue et ses parties latérales 17 symétriques, par rapport à un axe central 18.

La pièce fémorale se termine de l'autre côté par des parties d'extrémité 19, opposées à la partie en pointe de la surface trochléenne, lesdites parties 19 étant recourbées vers l'intérieur.

Dans ce mode de réalisation, ces deux parties 19 en vis à vis sont reliées par l'intermédiaire d'une poutre 20 centrale, de jonction, refermant la fente 8 de guidage du pion.

La fente 8 est par exemple de forme sensiblement rectangulaire présentant une courbure correspondant à celle de la pièce fémorale.

Dans le mode de réalisation plus particulièrement décrit ici, la fente 8 de largeur un peu supérieure à celle du pion 6, par exemple de 2 mm, présente une longueur par exemple égale aux deux tiers de la longueur déployée de la surface de coopération de la pièce fémorale 2 avec la plaque méniscale 4.

La plaque méniscale 4 comporte quant à elle une première partie 21 antérieure au pion 6 remontant vers l'extérieur, et une deuxième partie 22 postérieure au pion central de la plaque méniscale, munie d'une zone arrondie 23 de renfort, reliant les deux zones 10 postéro latérales, de frottement articulaire, de forme complémentaire aux deux zones latérales de la face externe 9 du bouclier trochléen de la pièce fémorale.

Cette zone 23 de renfort peut elle-même être munie d'un évidement 24 vers l'extérieur, situé sur sa surface externe, permettant une meilleure préhension de la plaque méniscale.

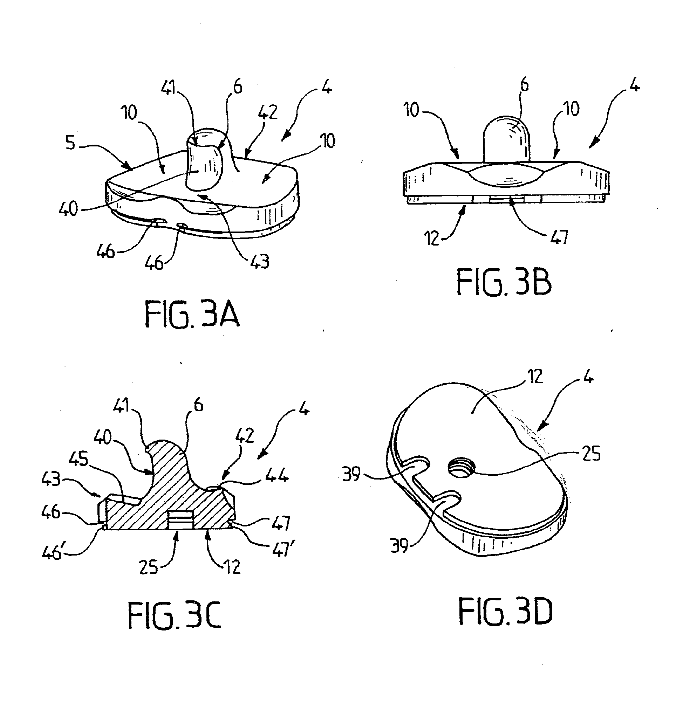

La plaque méniscale 4 va être plus particulièrement décrite ci-après en référence aux figures 3A à 3D.

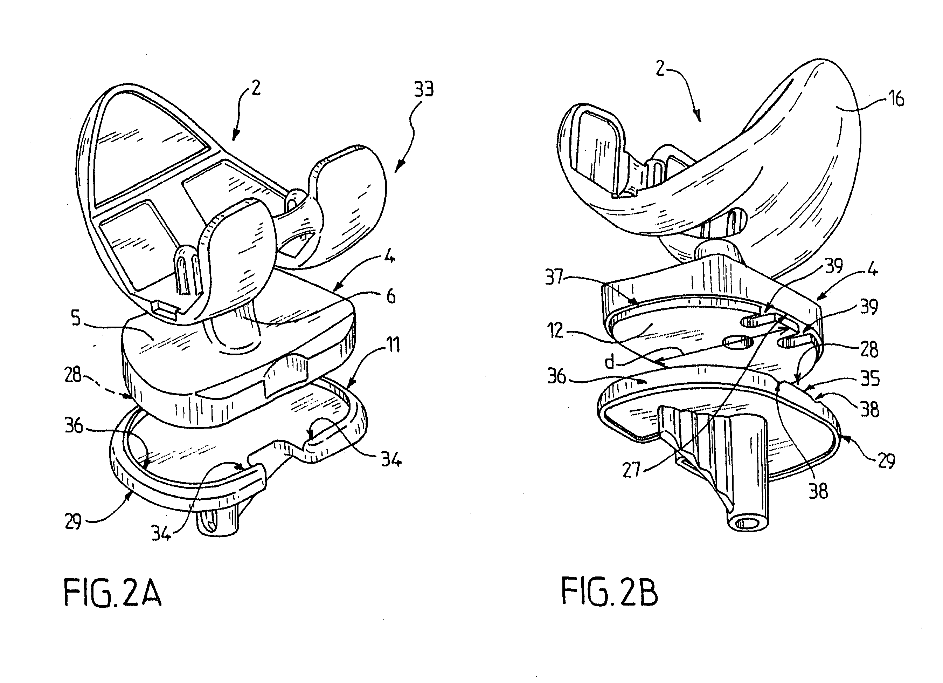

Mais d'ores et déjà notons que cette plaque méniscale comporte en combinaison un orifice central 25 (cf figure 1B) cylindrique, en forme de trou borgne, propre à venir s'encliquer pour coopérer à frottement doux avec un pion de pivotement 26 du plateau tibial 3 (cf figure 1A) de forme complémentaire cylindrique, et au moins un évidement 27 (cf figure 2B), de blocage propre à s'encastrer dans un doigt d'indexage 28 du plateau tibial 29 (cf figure 2B).

En référence aux figures 1A, 1B, 2A, 2B, il va maintenant être décrit les deux modes de réalisation de plateau tibial 3 ou 29 utilisable avec la prothèse selon l'invention.

Sur les figures 1A, 1B, il s'agit donc d'un plateau tibial 3 qui comporte un pion central 26 de pivotement situé sensiblement au centre de la face 11 de jonction tibiale, coopérant avec la face de jonction méniscale 12.

Le pion 26 est cylindrique et comporte par exemple une extrémité formant un léger ressaut qui vient s'encliquer dans une gorge complémentaire interne au trou borgne 25.

Le plateau tibial 3 comporte par ailleurs un pied d'ancrage 30 dans l'os, de forme connue en elle-même, par exemple constitué d'un plot central 31 muni de deux ailes latérales cannelées 32 formant un V, permettant une bonne fixation avec indexation dans l'os une fois que celui-ci a été coupé par le chirurgien pour venir y implanter ledit plateau tibial.

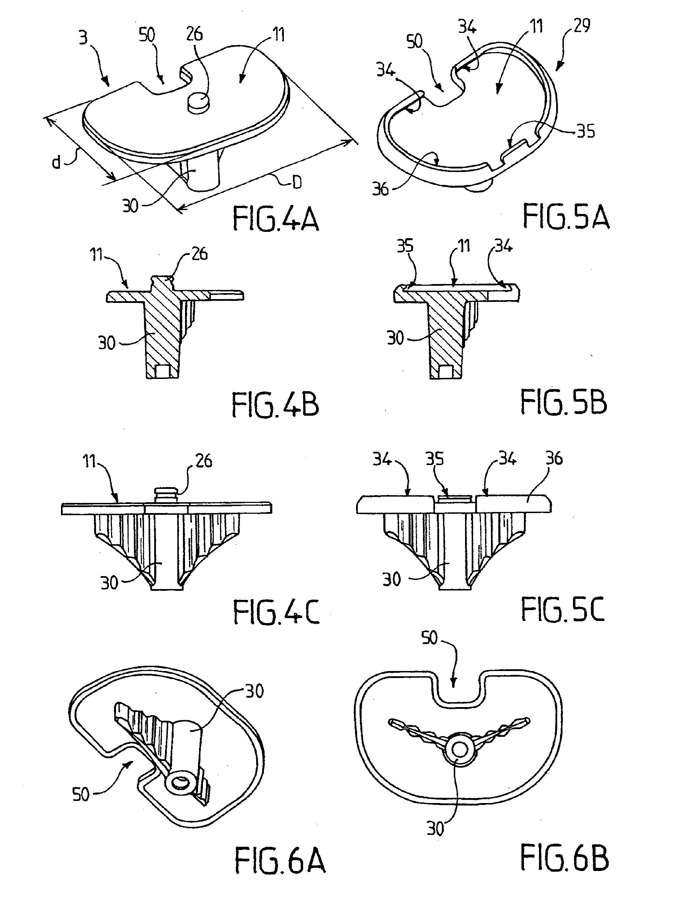

Les figures 2A et 2B montrent une prothèse 33 selon l'autre mode de réalisation de l'invention plus particulièrement décrit ici, comprenant le plateau tibial 29 qui présente une forme sensiblement similaire, mais cette fois-ci avec deux doigts d'indexage postérieurs 34 et un doigt d'indexage antérieur 35 (équivalent au doigt 28 mentionné ci-avant). Ces derniers apparaitront plus clairement en référence à la figure 5B décrite ci-après.

Dans le mode de réalisation de la figure 2A, les doigts antérieurs et postérieurs sont reliés par le bord périphérique 36 du plateau, bord surélevé qui, au niveau de la partie antérieure et au niveau de la partie postérieure de la prothèse, présente donc des configurations de doigt de clippage.

Mais le clippage peut avantageusement s'effectuer sur la totalité de la périphérie de plateau, auquel cas l'ensemble de la périphérie 36 s'enclippe avec un évidement en vis à vis 37 du bord de la face méniscale.

Il est par ailleurs prévu deux évidements 39 en languette disposée en épis par rapport à la périphérie de la face de jonction méniscale 12.

Un évidement en languette est par exemple de forme sensiblement ovale ou rectangulaire, de longueur correspondant au cinquième ou au septième de la distance d de la face méniscale. Ces évidements permettent, lorsqu'on y glisse un instrument, de faire levier et de déconnecter la plaque méniscale du plateau tibial quand il y a eu enclipsage.

Avantageusement le bord périphérique 36 du plateau est légèrement évidé en 38 au niveau des « languettes » pour faciliter l'introduction de l'outil formant bras de levier.

On a représenté plus précisément sur les figures 3A à 3D la plaque méniscale 4 qui vient d'être décrite en référence aux figures 1.

Cette plaque 4 comporte donc une face supérieure 5 munie d'un pion 6 en forme de bonnet phrygien ou d'extrémité de pouce, muni d'une partie 40 en cuvette terminée par une lèvre 41 supérieure formant un léger ressaut.

mode de réalisation, être démunie de pion 10, notamment Mais la plaque 4 peut bien entendu, dans un autre lorsque le ligament antéro-postérieur est conservé par le chirurgien.

La face supérieure 5 comprend de part et d'autre du côté antérieur 42 et postérieur 43 de la surface, des parties surélevées 44 et 45 par exemple de 1 à 2 mm par rapport à la surface adjacente. La face 5 comprend également de part et d'autre du pion, des surfaces de développement concaves formant les parties de guidage 10.

La face méniscale 12 de coopération avec la face tibiale 11 comporte (cf figure 3C et 3D) l'orifice de jonction 25 avec le pion 26 d'un plateau tibial, par exemple permettant un léger clippage du fait de la forme du pion, comme décrit ci-avant, et ensuite une rotation à frottement doux de l'un par rapport à l'autre.

La face 12 comporte également (cf figure 3C) des évidements 46, 47 agencés pour coopérer avec les doigts 34, 35 en vis à vis du plateau tibial, dans le cas où on souhaite que celle-ci soit bloquée.

Les évidements sont par exemple formés par des rainures avec excroissance 46', 47' dans le plan de la face de jonction 12.

Les deux modes de réalisation de plateaux tibiaux décrits en référence aux figures 1 et 2, sont quant à eux plus précisément représentés sur les figures 4A à 4C d'une part, 5A à 5C d'autre part, et 6A et 6B pour les vues de dessous.

Sur les figures 4A à 4C, le plateau 3 comporte le pion 26 permettant la rotation de la plaque méniscale par rapport au plateau.

Il s'agit donc d'un plateau rotatoire. La face de jonction tibiale 11 est de dimension antéro postérieure d et médio latérale D légèrement supérieure à la face méniscale 12 pour ne pas déborder de celle-ci lors de la rotation qui peut s'effectuer de quelques degrés.

Grâce aux dimensions relatives entre face de jonction méniscale et face de jonction tibiale, il va être possible d'autoriser une rotation sans débordement externe même en cas de mélange de tailles nécessaire du fait de contraintes anatomiques.

Par exemple, si on prend une pièce fémorale de taille 5, une plaque méniscale de taille 5 et un plateau tibial plus petit, d'une taille 4, les parties molles du genou restent encore protégées, même en cas de rotation de quelques degrés.

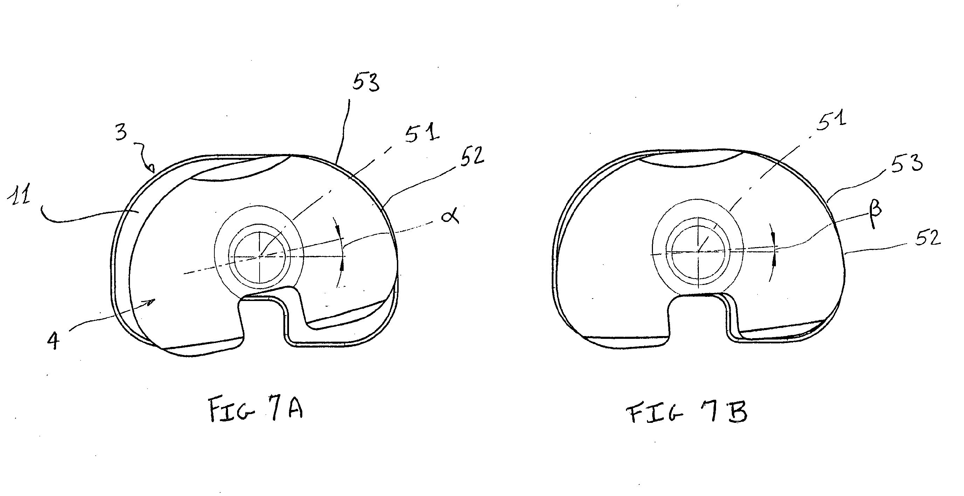

Cette caractéristique importante dans le mode de réalisation de l'invention plus particulièrement décrit ici, sera détaillée en référence aux figures 7A et 7B.

Le mode de réalisation de figures 5A à 5C décrit quant à lui un plateau pour plaque méniscale fixe.

Ici la plaque méniscale vient se clipper de manière rigide dans l'embase constituée par le plateau tibial 29, qui est ici encore de dimension légèrement supérieure.

Lorsqu'il s'agissait dans l'art antérieur d'aménager les tailles du fait de contraintes anatomiques, la différenciation se faisait surtout au niveau du fémur.

Avec l'invention qui permet de diminuer par deux le nombre de plaques méniscales, l'insert est toujours de dimension plus petite ce qui lui permet de s'adapter sur une embase aussi bien fixe que mobile. C'est donc l'embase fixe ou plateau tibial qui de par sa configuration particulière s'adapte à l'insert ou plaque méniscale.

Enfin avantageusement la face de jonction tibiale comprend une partie périphérique ajourée 50 de passage d'un doigt sous la face de jonction méniscale, permettant de le déconnecter du plateau. Cet évidement 50 est par exemple sensiblement rectangulaire, de largeur comprise entre 1/10^{e} et 1/3^{e} par exemple 1/4^{e} de la dimension d, et de longueur comprise entre 1/10^{e} et 1/3^{e} de D par exemple 1/5^{e}.

Les figures 7A et 7B montrent en vue de dessus, le plateau tibial 3 de la figure 1 sur lequel est enclippé la plaque méniscale 4. Celle-ci peut pivoter autour de l'axe 51 du pion 26, les faces de jonction 11 et 12 respectivement du plateau tibial et de la plaque méniscale coopérant à frottement doux. Avantageusement l'axe 51 du pion 26 coïncide avec l'axe du pion 6.

Lorsque la plaque méniscale pivote légèrement lors des mouvements du patient, autour de l'axe 51, on observe que le bord périphérique externe 52 de la plaque méniscale de section en forme d'ellipse tronquée reste à l'intérieur de la face de jonction 11, c'est à dire ne dépasse pas vers l'extérieur du bord périphérique 53, du plateau tibial pour un angle α de l'ordre de 12° lorsque, de façon normale, les pièces sont de même taille (figure 7A) ou pour un angle β de l'ordre de 4° lorsque le plateau tibial est d'une taille plus petite que la plaque méniscale

On va maintenant décrire en référence aux figures 1A et 1B la mise en place d'une prothèse selon l'invention par un chirurgien.

Lorsque ce dernier débute une opération, il détermine tout d'abord en fonction de la pathologie du patient ce qui correspond le mieux à sa configuration osseuse.

A partir de là il décide de poser une prothèse fixe ou une prothèse mobile.

Il choisit ensuite le plateau tibial correspondant à la solution fixe ou mobile.

Cette solution ayant été prise, il n'a plus ensuite qu'à mettre en place en fonction de la taille du patient la pièce fémorale qui est standard quelle que soit le type fixe ou mobile qui a été choisi. Il ne lui reste enfin qu'à déterminer quelle est la taille de la plaque méniscale en fonction du plateau tibial retenu et de la configuration du patient.

Alors que dans l'art antérieur il devait par exemple choisir parmi quatre vingt seize plaques méniscales différentes en fonction du plateau tibial retenu, il n'a plus aujourd'hui qu'à choisir parmi quarante huit plaques méniscales, grâce à l'invention.

Les gains comme on l'a indiqué tant en temps qu'en coûts notamment de stockage sont considérables.

Comme il va de soi et comme il résulte également de ce qui précède, la présente invention n'est pas limitée aux modes de réalisation plus particulièrement décrits. Elle en embrasse au contraire toutes les variantes et notamment celles où le plaques méniscales sont en matière plastique et celles où les surfaces de coopération entre le bouclier trochléen de la pièce fémorale et la plaque méniscale sont de configurations différentes, celles où la plaque méniscale ne comporte pas de pion sur sa face supérieure, notamment dans les cas de chirurgie où le ligament croisé n'est pas enlevé.

## Revendications

1. Prothèse (1, 33) du genou comprenant une pièce fémorale (2) reliée à un plateau tibial (3, 29) par une plaque méniscale (4) intermédiaire munie d'une face supérieure (5), ladite pièce fémorale (2) comportant un bouclier trochléen (7) muni d'une face externe (9) coopérant à frottement doux avec au moins une portée de guidage (10) de forme complémentaire ménagée dans ladite face supérieure, la plaque méniscale (4) intermédiaire et le plateau tibial (3, 29) étant reliés l'un à l'autre par l'intermédiaire de deux faces planes de jonction, à savoir une face de jonction tibiale (11) et une face de jonction méniscale (12), la face de jonction tibiale (11) étant plus grande que la face de jonction méniscale dans ses dimensions antéro postérieures (d) et médio latérales (D),
**caractérisée en ce que** le plateau tibial (3, 29) comprend un pion central de pivotement (26) ou au moins un doigt de clippage/indexage (34, 35, 36) périphérique, ledit pion ou ledit doigt étant en saillie par rapport à la face de jonction tibiale (11),
et **en ce que** la face de jonction méniscale (12) de la plaque méniscale (4) comprend en combinaison un orifice borgne central (25) propre à coopérer à frottement doux sur toute sa périphérie avec le pion (26) de pivotement du plateau tibial dans un cas et au moins un évidement périphérique (46, 47) de blocage propre à s'encastrer avec le doigt d'indexage (34, 35) du plateau tibial dans l'autre, selon que le plateau
tibial comporte un pion de pivotement ou un doigt d'indexage.

2. Prothèse selon la revendication 1, **caractérisée en ce que** les surfaces planes de jonction de la plaque méniscale avec le plateau tibial sont en forme d'ellipse ou sensiblement en forme d'ellipse tronquée sur un bord parallèlement au grand axe de ladite ellipse.

3. Prothèse selon l'une quelconque des revendications précédentes **caractérisée en ce que** la périphérie externe de la face de jonction méniscale est agencée pour être inscrite dans la surface de jonction de la plaque tibiale pour un angle α de rotation entre les deux faces compris entre - 12° et + 12° par rapport à l'axe médio-latéral de la partie méniscale.

4. Prothèse selon la revendication 3, **caractérisée en ce que** l'angle α est compris entre - 4° et + 4°.

5. Prothèse selon l'une quelconque des revendications précédentes, **caractérisée en ce que** la face supérieure de la plaque méniscale est munie d'un pion et de deux portées de guidage ménagées dans la face supérieure de part et d'autre dudit pion (16), le bouclier trochléen de la face fémorale comportant une fente (8) de guidage dudit pion.

6. Prothèse selon l'une quelconque des revendications précédentes, **caractérisée en ce que** le plateau tibial (29) comprend au moins un doigt d'indexage (34, 35), agencé pour s'encastrer dans l'évidemment (46, 47) de blocage de la plaque méniscale dans une position déterminée par rapport au plateau tibial.

7. Prothèse selon la revendication 6, **caractérisée en ce que** le plateau tibial comprend deux doigts d'indexage postérieurs (34) et un doigt d'indexage antérieur (35) propre à coopérer avec deux évidements postérieurs (46) et un évidement antérieur (47) en vis à vis de la plaque méniscale.

8. Prothèse selon l'une des revendications 1 à 5, **caractérisée en ce que** la face de jonction tibiale (11) comporte un rebord périphérique (36) formant doigt de clippage le long d'au moins une partie de la périphérie dudit plateau dans lequel s'encastre entièrement la face méniscale.

9. Prothèse selon l'une des revendications 1 à 5, **caractérisée en ce que** le plateau tibial (3) comprend un pion central (26) de pivotement, la plaque méniscale étant montée mobile en rotation par rapport au plateau tibial autour dudit pion.

10. Prothèse selon l'une quelconque des revendications précédentes, **caractérisée en ce que** le pion (6) de la face supérieure de la plaque méniscale a une forme de bonnet phrygien ou de pouce.

11. Prothèse selon la revendication 10, **caractérisé en ce que** le bonnet phrygien comporte une lèvre supérieure (41) formant un léger ressaut agencé pour engendrer un mouvement de recul de la pièce fémorale en cas de décoaptation supérieure à 1 mm de celle-ci par rapport à la plaque méniscale.

12. Prothèse selon l'une quelconque des revendications précédentes, **caractérisée en ce que** la face de jonction méniscale (12) comporte au moins un évidement (39) en épis par rapport à la périphérie de ladite face agencé pour permettre la déconnexion de la plaque méniscale d'avec le plateau tibial.

13. Prothèse selon l'une quelconque des revendications précédentes, **caractérisée en ce que** la zone postérieure (43) au pion central de la plaque méniscale comporte une partie de renfort (45) reliant les deux zones postéro-latérales de frottement articulaire.

14. Prothèse selon l'une quelconque des revendications précédentes, **caractérisée en ce que** la pièce fémorale (2) et le plateau tibial (3, 29) sont en alliage métallique et **en ce que** la plaque méniscale est en matériau plastique polymère.

15. Kit de prothèses selon l'une quelconque des revendications précédentes, **caractérisé en ce que** il comporte au moins un plateau tibial (29) pour plaque méniscale mixte (4) fixe et au moins un plateau tibial (3) pour plaque méniscale, et un jeu de plaques méniscales mixte (4) correspondantes de plusieurs épaisseurs.

## Patentansprüche

1. Knieprothese (1, 33) mit einem femoralen Teil (2), das mit einer Tibiaplatte (3, 29) durch eine dazwischen angeordnete, eine Oberseite (5) aufweisende Meniskusscheibe (4) verbunden ist, wobei das femorale Teil (2) einen Trochleaschild (7) umfasst, der mit einer Außenfläche (9) versehen ist, welche mit sanfter Reibung mit mindestens einer komplementär in der Oberseite ausgebildeten Führungsfläche (10) zusammenwirkt, wobei die dazwischen angeordnete Meniskusscheibe (4) und die Tibiaplatte (3, 29) durch zwei ebene Verbindungsflächen miteinander verbunden sind, und zwar durch eine tibiale Verbindungsfläche (11) und eine meniskale Verbindungsfläche (12), wobei die tibiale Verbindungsfläche (11) hinsichtlich der sagittalen Abmessungen (d) und der mediolateralen Abmessungen (D) größer ist als die meniskale Verbindungsfläche,
**dadurch gekennzeichnet,**
**dass**
- die Tibiaplatte (3, 29) einen mittigen Drehzapfen (26) oder mindestens einen umfänglichen Clip- bzw. Indexierfinger (34, 35, 36) umfasst, wobei der Zapfen bzw. der Finger über die tibiale Verbindungsfläche (11) hinausragt, und
- die meniskale Verbindungsfläche (12) der Meniskusscheibe (4) eine Kombination umfasst, zum einen aus einem mittigen Sackloch (25), das geeignet ist, auf seinem gesamten Umfang mit sanfter Reibung mit dem Drehzapfen (26) der Tibiaplatte zusammenzuwirken, und zum anderen aus mindestens einer umfänglichen Blockierausnehmung (46, 47), die geeignet ist, mit dem Indexierfinger (34, 35) der Tibiaplatte in Eingriff zu gelangen, je nachdem, ob die Tibiaplatte einen Drehzapfen oder einen Indexierfinger aufweist.

2. Prothese nach Anspruch 1,
**dadurch gekennzeichnet,**
**dass** die ebenen Flächen zur Verbindung der Meniskusscheibe mit der Tibiaplatte ellipsenförmig bzw. im Wesentlichen in Form einer an einem Rand parallel zur Längsachse der Ellipse abgestumpften Ellipse ausgebildet sind.

3. Prothese nach einem beliebigen der vorhergehenden Ansprüche,
**dadurch gekennzeichnet,**
**dass** der Außenumfang der meniskalen Verbindungsfläche derart angeordnet ist, dass er in die Tibiaplatte-Verbindungsfläche bei einem zwischen -12° und +12° zur mediolateralen Achse des meniskalen Teils liegenden Drehwinkel α der zwei Flächen zueinander eingeschrieben ist.

4. Prothese nach Anspruch 3,
**dadurch gekennzeichnet,**
**dass** der Winkel α zwischen -4° und +4° liegt.

5. Prothese nach einem beliebigen der vorhergehenden Ansprüche,
**dadurch gekennzeichnet,**
**dass** die Oberseite der Meniskusscheibe mit einem Zapfen und zwei Führungsflächen versehen ist, die in der Oberseite beidseitig des Zapfens (16) angeordnet sind, wobei der Trochleaschild der femoralen Fläche einen Schlitz (8) für die Führung des Zapfens aufweist.

6. Prothese nach einem beliebigen der vorhergehenden Ansprüche,
**dadurch gekennzeichnet,**
**dass** die Tibiaplatte (29) mindestens einen Indexierfinger (34, 35) aufweist, der so angeordnet ist, dass er in einer bestimmten Stellung in Bezug auf die Tibiaplatte mit der Blockierausnehmung (46, 47) der Meniskusscheibe in Eingriff gelangt.

7. Prothese nach Anspruch 6,
**dadurch gekennzeichnet,**
**dass** die Tibiaplatte zwei hintere Indexierfinger (34) und einen vorderen Indexierfinger (35) aufweist, welcher geeignet ist, mit zwei hinteren Ausnehmungen (46) und einer vorderen Ausnehmung (47) gegenüber der Meniskusscheibe zusammenzuwirken.

8. Prothese nach einem der Ansprüche 1 bis 5,
**dadurch gekennzeichnet,**
**dass** die tibiale Verbindungsfläche (11) einen umfänglichen Rand (36) aufweist, der einen Clipfinger entlang mindestens einem Teil des Umfangs der Platte bildet, mit der die Meniskusfläche vollständig in Eingriff gelangt.

9. Prothese nach einem der Ansprüche 1 bis 5,
**dadurch gekennzeichnet,**
**dass** die Tibiaplatte (3) einen mittigen Drehzapfen (26) umfasst, wobei die Meniskusscheibe drehbeweglich um den Zapfen in Bezug auf die Tibiaplatte montiert ist.

10. Prothese nach einem beliebigen der vorhergehenden Ansprüche,
**dadurch gekennzeichnet,**
**dass** der Zapfen (6) der Oberseite der Meniskusscheibe die Form einer phrygischen Mütze oder eines Daumens aufweist.

11. Prothese nach Anspruch 10,
**dadurch gekennzeichnet,**
**dass** die phrygische Mütze eine obere Lippe (41) aufweist, die einen leichten Rücksprung bildet, welcher derart angeordnet ist, dass bei Dekoaptation des femoralen Teils um mehr als 1 mm in Bezug auf die Meniskusscheibe eine Rückwärtsbewegung desselben verursacht wird.

12. Prothese nach einem beliebigen der vorhergehenden Ansprüche,
**dadurch gekennzeichnet,**
**dass** die meniskale Verbindungsfläche (12) mindestens eine quer zum Umfang der Fläche ausgebildete Ausnehmung (39) aufweist, welche derart angeordnet ist, dass die Meniskusscheibe von der Tibiaplatte getrennt werden kann.

13. Prothese nach einem beliebigen der vorhergehenden Ansprüche,
**dadurch gekennzeichnet,**
**dass** der in Bezug auf den mittigen Zapfen der Meniskusscheibe hintere Bereich (43) einen Verstärkungsabschnitt (45) aufweist, welcher die beiden posterolateralen Gelenkreibungsbereiche verbindet.

14. Prothese nach einem beliebigen der vorhergehenden Ansprüche,
**dadurch gekennzeichnet,**
**dass** das femorale Teil (2) und die Tibiaplatte (3, 29) aus einer metallischen Legierung bestehen und die Meniskusscheibe aus einem Polymerkunststoff besteht.

15. Prothesenbausatz nach einem beliebigen der vorhergehenden Ansprüche,
**dadurch gekennzeichnet,**
**dass** er mindestens eine Tibiaplatte (29) für eine ortsfeste Kombi-Meniskusscheibe (4) und mindestens eine Tibiaplatte (3) für Meniskusscheibe sowie einen Satz entsprechender, unterschiedlich dicker Kombi-Meniskusscheiben (4) aufweist.

## Claims

1. Knee prosthesis (1, 33) comprising a femoral part (2) connected to a tibial plateau (3, 29) by an intermediate meniscal plate (4) provided with an upper face (5), said femoral part (2) comprising a trochlear shield (7) provided with an external face (9) cooperating with mild friction with at least one guiding bearing surface (10) of complementary shape formed in said upper face, the intermediate meniscal plate (4) and the tibial plateau (3, 29) being connected to one another through the intermediary of two planar junction faces, namely a tibial junction face (11) and a meniscal junction face (12), the tibial junction face (11) being larger than the meniscal junction face in its anteroposterior dimensions (d) and its mediolateral dimensions (D),
**characterised in that** the tibial plateau (3, 29) comprises a central pivot pin (26) or at least one peripheral clipping/indexing finger (34, 35, 36), said pin or said finger projecting in relation to the tibial junction face (11),
and **in that** the meniscal junction face (12) of the meniscal plate (4) comprises in combination a central blind hole (25) capable of cooperating with mild friction over its entire periphery with the pivot pin (26) of the tibial plateau in the first case and at least one peripheral locking recess (46, 47) capable of engaging with the indexing finger (34, 35) of the tibial plateau in the other case, depending on whether the tibial plateau has a pivot pin or an indexing finger.

2. Prosthesis according to claim 1, **characterised in that** the planar junction surfaces of the meniscal plate with the tibial plateau are in the shape of an ellipse or substantially in the shape of an ellipse which is truncated on an edge parallel to the main axis of said ellipse.

3. Prosthesis according to any one of the preceding claims, **characterised in that** the external periphery of the meniscal junction face is arranged to be inscribed within the junction surface of the tibial plateau for an angle α of rotation between the two faces of between -12° and +12° in relation to the mediolateral axis of the meniscal part.

4. Prosthesis according to claim 3, **characterised in that** the angle α is between -4° and +4°.

5. Prosthesis according to any one of the preceding claims, **characterised in that** the upper face of the meniscal plate is provided with a pin and two guiding bearing surfaces formed in the upper face on either side of said pin (16), the trochlear shield of the femoral face having a slot (8) for guidance of said pin.

6. Prosthesis according to any one of the preceding claims, **characterised in that** the tibial plateau (29) comprises at least one indexing finger (34, 35), arranged to engage in the locking recess (46, 47) of the meniscal plate in a given position in relation to the tibial plateau.

7. Prosthesis according to claim 6,**characterised in that** the tibial plateau comprises two posterior indexing fingers (34) and one anterior indexing finger (35) capable of cooperating with two posterior recesses (46) and one anterior recess (47) facing the meniscal plate.

8. Prosthesis according to one of claims 1 to 5, **characterised in that** the tibial junction face (11) comprises a peripheral rim (36) forming a clipping finger along at least a part of the periphery of said plateau in which the meniscal face is embedded entirely.

9. Prosthesis according to one of claims 1 to 5, **characterised in that** the tibial plateau (3) comprises a central pivot pin (26), the meniscal plate being mounted so that it is movable in rotation in relation to the tibial plateau around said pin.

10. Prosthesis according to any one of the preceding claims, **characterised in that** the pin (6) of the upper face of the meniscal plate has the shape of a Phrygian cap or thumb.

11. Prosthesis according to claim 10, **characterised in that** the Phrygian cap has an upper lip (41) forming a slight bulge arranged to produce a backward movement of the femoral part in the event of decoaptation of the latter by more than 1 mm in relation to the meniscal plate.

12. Prosthesis according to any one of the preceding claims, **characterised in that** the meniscal junction face (12) comprises at least one recess (39) which is arranged diagonally in relation to the periphery of said face so as to allow the disconnection of the meniscal plate from the tibial plateau.

13. Prosthesis according to any one of the preceding claims, **characterised in that** the posterior zone (43) of the central pin of the meniscal plate comprises a reinforcing part (45) connecting the two posterolateral zones of articular friction.

14. Prosthesis according to any one of the preceding claims, **characterised in that** the femoral part (2) and the tibial plateau (3, 29) are made of a metal alloy and **in that** the meniscal plate is made of a polymeric plastic material.

15. Kit for prostheses according to any one of the preceding claims, **characterised in that** it comprises at least one tibial plateau (29) for a fixed mixed meniscal plate (4) and at least one tibial plateau (3) for a meniscal plate, and a set of corresponding mixed meniscal plates (4) in a plurality of thicknesses.
